# EUROPEAN PATENT APPLICATION

(11) **EP 1 835 032 A1**
(43) Date of publication of application: **19.09.2007**
(21) Application number: 06111064.9
(22) Date of filing: 14.03.2006
(51) Int. Cl.: C12N 15/867

(54) **A self-inactivating recombinant lentiviral vector for the inhibition of HIV replication**

(71) Applicant: Université de Liège, 4020 Liège (BE)
(72) Inventor: Ravet, Emmanuel Université de Liège, 4000 Liège (BE); Brule-Ravet, Fabienne Université de Liège, 4000 Liège (BE); Khatissian, Emmanuel Université de Liège, 4000 Liège (BE); Piette, Jacques Université de Liège, 4000 Liège (BE)
(74) Representative: polypatent

(57) **Abstract**

The present invention refers to a self-inactivating recombinant lentiviral vector comprising a 5' LTR and a 3' LTR, wherein the LTR region has been rendered substantially transcriptionally inactive by virtue of deletions in the U3 region of the 3' LTR, characterized in that the lentiviral vector is further comprising a beta-interferon gene positioned under the control of a promoter.

## Description

The present invention refers to a self-inactivating recombinant lentiviral vector having the sequences cPPT and CTS and an expression cassette comprising a transgene for the inhibition of HIV replication.

Despite numerous ways of research and an abundant number of new treatments, infection with HIV and AIDS continues to be a worldwide health problem. The current anti-retroviral combination drug therapy used in human immunodeficiency virus type 1 (HIV-1)-infected individuals, named HAART (Highly Active Anti-retroviral Therapy) reduces morbidity and mortality by reducing viral load, enhancing the number of CD4⁺ cells hence restoring immunity ¹. However, this therapy presents several drawbacks because of the time and difficulty to completely purge the viral reservoir ². On the other hand, the toxicity of HAART is clearly established ^{3,4}, and the number of failures increase due to the difficulty to obtain a good adhesion to this lifetime treatment ⁵ and to the evolution of antiretroviral resistance in the face of drug pressure, which is characterized by the apparition of numerous resistant variants ^{6,7}.

Alternative strategies to inhibit virus replication, either alone or in combination with those currently practiced, are required to eradicate the virus. Gene therapy protocols offer a good opportunity to obtain a better control of the disease. Previous studies have used transdominant proteins ^{8,9}, RNA decoys, ribozyme, antisense RNA ¹⁰⁻¹⁴ and interfering RNA (iRNA) ¹⁵⁻¹⁷. While a large part of these studies have used viral targets or cellular co-factors, few of them have exploited innate cellular mechanism. A well-described mechanism that may restrict HIV replication is the pleiotropic antiviral effect of interferon (IFN)-β which is known to affect various stages of the HIV cycle life from uptake of the viral particles to the release of the newly formed virions ¹⁹⁻²⁵.

Several studies have demonstrated that moderate and continuous IFN-β production obtained in human PBMC and CD4⁺ cells transduced with a MoMuLV-derived vector can reduce the *in vitro* HIV replication ^{19,26}. However, reduction of *in vitro* HIV replication was only from 1.3 to 31 fold in CD4⁺ cells. In addition, IFN-β contributes to restore normal immune cell functions by enhancing IFN-γ and IL-12 production and by returning IL-4, IL-6, IL-10 and TNF-α production to normal levels. Moreover, the cytotoxic response of CD8⁺ cells against HIV-infected cells is also improved ^{26,27}. *In vivo* studies have shown that IFN-β transduced human CD4⁺ cells transferred into a hu-PBL-SCID mouse model, which supports constitutive HIV replication, displayed a reduced HIV replication and an enhanced CD4⁺ cell survival ²⁸. Similar experiments performed in the macaque model have only shown a transient presence of a small number of cells producing IFN-β and no particular effect on SIVmac251 viremia. These results were expected considering the low transduction efficiency of the MoMuLV-derived vector and the small number of transduced cells infused into the animals ²⁹. The drawback is the low transduction efficiency of the prior art constructs and that only proliferating cells could be transduced.

Further, in the prior art, it is well known that lentiviral-derived vectors are able to transduce haematopoietic cells at high levels, including progenitors and CD4⁺ cells ^{30,31}.

The object of the present invention was to provide DNA vectors for gene therapy which further increase the inhibition of HIV replication with higher efficiency.

The object of the present invention was solved by a self-inactivating recombinant lentiviral vector (SIN) comprising a 5' LTR and a 3' LTR, wherein the LTR region has been rendered substantially transcriptionally inactive by virtue of deletions in the U3 region of the 3' LTR, wherein the lentiviral vector further comprises a beta-interferon gene positioned under the control of a promoter. Preferably, the lentiviral vector comprises at least the following elements: 5' LTR, PBS (primer binding site), Psi (ψ)-site, RRE (Rev Responsive Element), cPPT and CTS sequence, the promoter for expression of the beta-interferon gene, the beta-interferon gene, and 3' LTR.

The lentiviral vector is capable of transducing non-dividing (quiescent) cells. Therefore, lentiviral vectors according to the present invention are especially useful to transduce cells selected from the group consisting of CD4⁺ cells, PBMC, macrophages, CD34⁺ cells and haematopoietic stem cells in order to integrate the beta-interferon gene into the chromosome of these cells. As a result, the beta-interferon will be expressed from the integrated form of the lentiviral vector sequence and will result in the inhibition of HIV replication. These transduced cells can then be transferred into the human patient, for therapeutic treatment of HIV infection.

Hereinafter, the term "lentiviral vector" will be used to denote any form of a nucleic acid derived from a lentivirus and used to transfer genetic material into a cell via transduction. The term encompasses lentiviral vector nucleic acids, such as DNA and RNA, encapsidated forms of these nucleic acids, and viral particles in which the viral vector nucleic acids have been packaged. The sequence of the lentiviral vector can be comprised in a plasmid, which is used to produce lentiviral vector nucleic acid by transcription of RNA molecules after the plasmid has been transfected into a cell. If this cell is a viral particle producer cell, namely if the cell is also co-transduced with an encapsidation plasmid and a vesicular stomatitis virus envelope expression plasmid, viral particles can be produced which contain the lentiviral vector nucleic acid (in form of RNA) as vector genome. According to the invention, these viral particles (containing the lentiviral vector RNA comprising a beta-interferon gene positioned under the control of a promoter flanked by 5' LTR and 3' LTR) are subsequently used to transduce cells selected from the group of CD4⁺ cells, monocytes, PBMC, macrophages, CD34⁺ cells and haematopoietic stem cells, in order to integrate the lentiviral vector sequence (comprising a beta-interferon gene positioned under the control of a promoter flanked by 5' LTR and 3' LTR) into the chromosome of these cells. As a result, the beta-interferon will be expressed from the integrated form of the lentiviral vector sequence and will result in the inhibition of HIV replication.

In a preferred embodiment the promoter is active to promote detectable transcription of the beta-interferon gene in human cells presenting a target for human immunodeficiency virus.

In a further preferred embodiment the human cells presenting a target for human immunodeficiency virus are selected from the group consisting of CD4⁺ cells, monocytes, peripheral blood mononuclear cells (PBMC) and macrophages, most preferred T lymphocytes CD4⁺ and PBMC.

It is further preferred that the promoter is active to promote detectable transcription of the beta-interferon gene in cells selected from the group consisting of CD4⁺ cells, monocytes, PBMC, macrophages, CD34⁺ cells and haematopoietic stem cells, most preferred T lymphocytes CD4⁺ and PBMC. Preferably the promoter is a ubiquitous promoter and also a comparably weak promoter. The activity of the promoter must not be too strong so that over-expression of the transgene (beta-interferon gene) and by that the risk of cytotoxicity is avoided. Suitable promoters are H2-kappa-B, CMV and EF1alpha. The H2-kappa-B promoter is most preferred. Preferably the vector contains a murine H2-kappa-B promoter.

In a preferred embodiment the LTR regions have reduced promoter activity compared to the wild-type LTR. This preferred embodiment makes sure that the beta-interferon gene is expressed only by the H2-kappa-B promoter or other suitable promoters and minimizes the risk of insertional oncogenesis.

Furthermore, it is preferred that the vector is incapable of reconstituting a wild-type lentivirus through recombination.

It is preferred that the deletion in the U3 region (deltaU3) of 3' LTR includes a TATA box sequence. In a preferred embodiment the U3 region of 3' LTR deleted is all of said U3 region except for a 5' terminal dinucleotide (sequence specifically cleaved during the 3' processing process) and an att (recognition site for the integrase protein) sequence.

Further, the present invention provides a plasmid, comprising the sequence of the lentiviral vector according to the present invention. The vector plasmid is carrying the sequences of the lentiviral vector. If the vector is transduced into a cell, the sequences of the lentiviral vector will be transcribed, wherein a RNA molecule is produced which is representing the lentiviral vector genome. If the cell is a viral particle producer cell, the lentiviral vector genome will be contained in the viral particle.

The object of the present invention is also solved by the use of the lentiviral vector according to the present invention for transducing cells selected from the group consisting of CD4⁺ cells, monocytes, PBMC, macrophages, CD34⁺ cells and haematopoietic stem cells, most preferred T lymphocytes CD4⁺ and PBMC. The present invention therefore provides the use of a self-inactivating recombinant lentiviral vector comprising a 5' LTR and a 3' LTR, wherein the LTR region has been rendered substantially transcriptionally inactive by virtue of deletions in the U3 region of the 3' LTR, wherein the lentiviral vector is further comprising a beta-interferon gene positioned under the control of a promoter, for transducing cells selected from the group consisting of CD4⁺ cells, monocytes, PBMC, macrophages, CD34⁺ cells and haematopoietic stem cells, most preferred T lymphocytes CD4⁺ and PBMC.

The object is further solved by a cell having the lentiviral vector according to the present invention stably integrated into its chromosomal DNA. Preferably, the cell is selected from the group consisting of CD4⁺ cells, monocytes, PBMC, macrophages, CD34⁺ cells and haematopoietic stem cells, most preferred T lymphocytes CD4⁺ and PBMC.

The present invention further provides the use of the lentiviral vector of the present invention for therapeutic and/or preventive treatment of HIV infection. Further, it is provided the use of the lentiviral vector of the present invention for the inhibition of the replication of HIV.

The object is further solved by the provision of a medicament comprising the lentiviral vector according to the present invention. The present invention therefore provides a medicament comprising a self-inactivating recombinant lentiviral vector comprising a 5' LTR and a 3' LTR, wherein the LTR region has been rendered substantially transcriptionally inactive by virtue of deletions in the U3 region of the 3' LTR, wherein the lentiviral vector is further comprising a beta-interferon gene positioned under the control of a promoter.

The present invention also provides the use of the lentiviral vector according to the present invention for the manufacture of a medicament for the therapeutic and/or preventive treatment of HIV infection. Further it is provided the use of the lentiviral vector of the present invention for the manufacture of a medicament for the inhibition of the replication of HIV.

Further it is provided a method for the transduction of cells comprising the step of transduction of cells with the vector of the present invention, wherein the cells are selected from the group consisting of CD4⁺ cells, monocytes, PBMC, macrophages, CD34⁺ cells and haematopoietic stem cells, most preferred T lymphocytes CD4⁺ and PBMC.

The present invention also provides a method for the preventive and/or therapeutic treatment of a human individual comprising the following steps:
- transduction of cells with a self-inactivating recombinant lentiviral vector comprising a 5' LTR and a 3' LTR, wherein the LTR region has been rendered substantially transcriptionally inactive by virtue of deletions in the U3 region of the 3' LTR, wherein the lentiviral vector is further comprising a beta-interferon gene positioned under the control of a promoter, wherein the cells are selected from the group consisting of CD4⁺ cells, monocytes, PBMC, macrophages, CD34⁺ cells and haematopoietic stem cells, most preferred T lymphocytes CD4⁺ and PBMC; and
- administration of the transduced cells into the human individual.

According to the present invention a lentiviral vector has been provided carrying the gene encoding human or simian beta-interferon, named LT-huIFN and LT-siIFN, respectively, under the control of a 0.6 kb fragment of the murine H2-Kb gene promoter, and culture conditions that permit to obtain high levels of gene transfer in CD4⁺ human cells and constant expression of interferon in transduced cells. In order to evaluate the effects of the interferon expression, phenotypic analyses of transduced cells have been performed without detectable side effects on CD4⁺ proliferation and apoptosis. Using these tools, heterologous IFN-β expression has been obtained in the CEMx174 cell line and in CD4⁺ and PBMC human cells. This exogenous IFN-β expression results in an extremely strong inhibition of the replication of different strains of HIV (X4 class and HIV-2 ROD) and SIV in the CEMx174 cell line and in primary cells. Expression of hulFN-β in CD4⁺ isolated cells or in total PBMC cells also leads to a radical inhibition of HIV replication, of up to 99.9%. It was also demonstrated that LT-huIFN transduced are unable to properly produce the P24 HIV protein and that interferon expression leads to a persistence of the CD4 expression with an augmented proliferation capacity.

A. Lentiviral Vectors and Gene Therapy.

The present invention is directed to the development of improved lentiviral vectors that meet biosafety requirements and induce sufficient and reasonable high levels of transduction efficiencies. Accordingly, the present invention describes gene transfer vehicles that appear particularly well suited for the transduction of CD4⁺ cells, PBMC, macrophages, CD34⁺ cells and haematopoietic stem cells and for the expression of transgenes in differentiated blood lineages.

Lentiviruses are a subgroup of retroviruses that can infect non-dividing cells owing to the karyophilic properties of the proteins integrated in the pre-integration complex, which allow for their import through the nucleopore³². Correspondingly, lentiviral vectors derived from human immunodeficiency virus type 1 (HIV-1) can mediate the efficient delivery, integration and long-term expression of transgenes into non-mitotic cells both *in vitro* and *in vivo.* In particular, HIV-based vectors can efficiently transduce human CD34⁺ haematopoietic cells in the absence of cytokine stimulation. Lentiviral vectors provide a basis for the gene therapy for example of acquired lympho-haematopoietic disorders.

The lentiviral vectors of the present invention provide an efficient mean of achieving heterologous expression of desired transgenes in CD4⁺ cells, PBMC, macrophages, CD34⁺ cells and haematopoietic stem cells. These lentiviral vectors have the ability to infect non-dividing cells owing to the karyophilic properties of their pre-integration complex, which allow for its active import through the nucleopore. Moreover, preferably the lentiviral vectors of the present invention can mediate the efficient delivery, integration and long-term expression of IFN-β into non-mitotic cells both *in vitro* and *in vivo,* even in the absence of cytokine stimulation. Most notably, the lentiviral vectors of the present invention permit the expression of IFN-β in human CD4⁺ cells, PBMC, macrophages, CD34⁺ cells and haematopoietic stem cells while meeting human biosafety requirements.

The most preferred lentiviral genes and backbone (*i*.*e*., long terminal repeats or LTRs) used in preparing lentiviral vectors in accordance with the present invention will be those that are human immunodeficiency virus (HIV)-derived, and more particularly, HIV-1 derived. Thus, the gag, pol, tat and rev genes will preferably be HIV genes and more preferably HIV-1 genes. However, the gag, pol, tat and rev genes and LTR regions from other lentiviruses may be employed for certain applications in accordance with the present invention, including the genes and LTRs of HIV-2, simian immunodeficiency virus (SIV), feline immunodeficiency virus, bovine immunodeficiency virus, equine infectious anaemia virus, caprine arthritis encephalitis virus and the likes. Such constructs could be useful, for example, where one desires to modify certain cells of non-human origin. However, the HIV-based vector backbones (*i.e.,* HIV LTR and HIV gag, pol and rev genes) will generally be preferred in connection with most aspects of the present invention in that HIV-based constructs are the most efficient to transduce human haematopoietic progenitor cells.

The viral vectors of the present invention will also include an expression cassette comprising the IFN-β gene positioned under the control of a promoter that is active to promote detectable transcription of the IFN-β gene in human CD4⁺ cells, PBMC, macrophages, CD34⁺ cells and haematopoietic stem cells, respectively. To determine whether a particular promoter is useful, a selected promoter is tested in the construct *in vitro* in a selected cell type and, if the promoter is capable of promoting expression of the transgene at a detectable signal-to-noise ratio, it will generally be useful in accordance with the present invention. A desirable signal-to-noise ratio is one between about 10 and about 200, a more desirable signal-to-noise ratio is one between 40 and about 200, and an even more desirable signal-to-noise ratio is one between about 150 and about 200. One means of testing such a promoter, described in more detail herein below, is through the use of a signal-generating transgene such as the enhanced green fluorescent protein (EGFP). Markers of transduction could also be YFP, RFP, CFP and other intrinsically fluorescent proteins. In another embodiment, cell surface proteins such as clusters of differentiation could be used as markers of transduction.

Examples of promoters that may be preferably employed in connection with the present invention including H2-kappa-B promoter. In any event, however, practice of the present invention is not restricted to the foregoing promoter, so long as the promoter is active in the CD4⁺ cells, PBMC, macrophages, CD34⁺ cells and haematopoietic stem cells that one desires to target.

Preferably, the present invention is directed to host cells that have been transduced with the foregoing lentiviral vector of the present invention. The lentiviral vectors of the present invention can be employed to transduce CD4⁺ T cell, a peripheral blood lymphocyte cell), a peripheral blood mononuclear cell (PBMC), a macrophage, or a haematopoietic progenitor/stem cell. The cells transduced may further be primate, murine, porcine, or human in origin, or come from another animal species.

For the production of virus particles, one may employ any cell type that is compatible with the expression of lentiviral gag and pol genes, or any cell type that can be engineered to support such expression. For example, producer cells such as 293T cells and HT1080 cells may be used.

In still other embodiments, the present invention is directed to a method for transducing human CD4⁺ cells, monocytes, PBMC, macrophages, CD34⁺ cells and haematopoietic stem cells, most preferred T lymphocytes CD4⁺ and PBMC, comprising contacting a population of human cells that include said cells with the lentiviral vectors of the present invention under conditions to effect the transduction of human CD4⁺ cells, monocytes, PBMC, macrophages, CD34⁺ cells and haematopoietic stem cells, most preferred T lymphocytes CD4⁺ and PBMC, in said population by the vector. The cells may be transduced *in vivo* or *in vitro,* depending on the ultimate application. Even in the context of human gene therapy, one may transduce the stem cell *in vivo* or, alternatively, transduce them *in vitro* followed by infusion of the transduced stem cell into a human subject. In one aspect of this embodiment, the human cell can be removed from a human, e.g., a human patient, using methods well known to those of skill in the art and transduced as noted above. The transduced cells are then reintroduced into the same or a different human.

Where a human subject is treated directly by introduction of the vector into the subject, the treatment is typically carried out by intravenous administration of the vector. When cells, for instance CD34⁺ cells, dendritic cells, peripheral blood cells or tumour cells are transduced *ex vivo,* the vector particles are incubated with the cells using a dose generally in the order of between 0.1 to 100 multiplicities of infection (MOI). This of course includes amount of vector corresponding to 0.1, 1, 2, 3, 4, 5, 6, 7, 8, 9, 10, 15, 20, 25, 30, 35, 40, 45, 50, 55, 60, 65, 70, 75, 80, 85, 90, 95 and 100 MOI. Typically, the amount of vector may be expressed in terms of transducing units (TU).

Lentiviruses are complex retroviruses, which, in addition to the common retroviral genes gag, pol, and env, contain other genes with regulatory or structural function including vif, vpr, tat, rev, vpu, nef and vpx (see for example U.S. Pat. NOs. 6,013,516 and 5,994,136). The higher complexity enables the virus to modulate its life cycle, as in the course of latent infection. Some examples of lentivirus include the Human Immunodeficiency Viruses: HIV-1, HIV-2 and the Simian Immunodeficiency Virus: SIV. The lentiviral genome and the proviral DNA have the three genes found in retroviruses: gag, pol and env, which are flanked by two long terminal repeat (LTR) sequences. The gag gene encodes the internal structural proteins (matrix, capsid and nucleocapsid); the pol gene encodes the RNA-directed DNA polymerase (reverse transcriptase), a protease and an integrase; and the env gene encodes viral envelope glycoproteins. The 5' and 3' LTR serve to promote transcription and polyadenylation of the virion's RNA. The LTR contains all other cis-acting sequences necessary for viral replication.

Adjacent to the 5' LTR are sequences necessary for reverse transcription of the genome (the tRNA primer binding site) and for efficient encapsidation of viral RNA into particles (the Psi "ψ" site). If the sequences necessary for encapsidation (or packaging of retroviral RNA into infectious virions) are missing from the viral genome, this cis defect prevents the encapsidation of genomic RNA.

Further essential elements in the vectors of the invention are the cPPT and CTS sequences: HIV-1 has evolved a complex reverse transcription strategy, which differs from that of oncoviruses by two steps occurring at the centre of the genome: one additional initiation of plus strand synthesis coupled with a termination step. HIV-1 plus strand DNA is synthesized as two discrete half-genomic segments. An additional copy of the polypurine tract cis-active sequence, present at the centre of all lentiviral genomes (cPPT), initiates synthesis of a downstream plus strand. The upstream plus strand segment initiated at the 3'PPT will, after a strand transfer, proceed to the centre of the genome and terminate after a discrete strand displacement event. This last chronological event of HIV-1 reverse transcription is controlled by the central termination sequence (CTS) cis-active sequence, which ejects HIV-1 reverse transcriptase (RT) at this site in the specific context of strand displacement synthesis. Thus the final product of the HIV-1 reverse transcription is a linear DNA molecule bearing in its centre a stable 99 nucleotide long DNA flap, here referred to as the central DNA triplex (see, US Patent Application NO. 2003/0194392).

Lentivirus-derived vectors are useful for gene therapy. They integrate stably into chromosomes of target cells, which is a pre-requisite for long-term expression. Additionnally, lentiviruses, in contrast to other retroviruses, are capable of transducing non-dividing cells. This is very important in the context of gene-therapy for tissues such as the haematopoietic system, in particular for the transduction of CD4⁺ cells, PBMC, macrophages, CD34⁺ cells and haematopoietic stem cells. Lentiviral vectors have been generated by multiply deletions of the HIV virulence genes, for example, the genes env, vif, vpr, vpu and nef are deleted making the vector biologically safe. Further, they do not transfer viral genes, therefore avoiding the problem of generating transduced cells that can be destroyed by cytotoxic T-cells. Preferably the lentiviral vectors of the present invention do also not contain active gag, pol and env genes. As mentioned before for viral particle production these genes can be provided on a separate plasmid.

Two components are involved in making a virus-based gene delivery system: first, the packaging elements, encompassing the structural proteins as well as the enzymes necessary to generate an infectious particle, and second, the vector itself, *i.e.,* the genetic material to be transferred. Biosafety safeguards can be introduced in the design of both of these components. Thus, the packaging unit of the first generation HIV-based vectors comprised all HIV-1 proteins except the envelope proteins. Subsequently it was shown that the deletion of four additional viral genes that are responsible for virulence including vpr, vif, vpu and nef did not alter the utility of the vector system. It was also shown that tat, the main transactivator of HIV is also dispensable for the generation of a fully efficient vector. Thus, the third-generation packaging unit of the HIV-based lentiviral vectors comprises only three genes of the parental virus: gag, pol and rev, thus limiting the possibility of reconstitution of a wild-type virus through recombination. This system was further improved by removing HIV transcriptional units from the vector. It was demonstrated that introducing a deletion in the U3 region of the 3' LTR of the DNA used to produce the vector RNA generated self-inactivating (SIN) vectors. During reverse transcription this deletion is transferred to the 5' LTR of the proviral DNA. Enough sequence was eliminated, including the removal of a TATA box, which abolished the transcriptional activity of the LTR, which prevents production of full-length vector RNA in transduced cells. This however did not affect vector titres or the *in vitro* or *in vivo* properties of the vector.

Introducing the lentiviral vector of the present invention carrying the beta-interferon gene, into a packaging cell yields a producer cell which releases infectious viral particles carrying the beta-interferon gene.

The packaging cell is further transfected with a plasmid carrying the env gene. The env gene can be derived from any virus, including retroviruses. The env preferably is the Vesicular stomatitis virus (VSV) protein G (VSV G) which allows transduction of cells of human and other species without the need of a surface receptor. Moreover an amphotropic envelope protein could be used. Examples of retroviral-derived env genes include, but are not limited to: Moloney murine leukemia virus (MoMuLV or MMLV), Harvey murine sarcoma virus (HaMuSV or HSV), murine mammary tumour virus (MuMTV or MMTV), gibbon ape leukaemia virus (GaLV or GALV), human immunodeficiency virus (HIV) and Rous sarcoma virus (RSV). Other env genes such as those of hepatitis and influenza viruses also can be used.

The vector providing the viral env nucleic acid sequence is operably associated with regulatory sequences, e.g., a promoter or enhancer. The regulatory sequence can be any eukaryotic promoter or enhancer, including for example, EF1.alpha., PGK, the Moloney murine leukaemia virus promoter-enhancer element, the human cytomegalovirus enhancer, the vaccinia P7.5 promoter or the likes. In some cases, such as the Moloney murine leukaemia virus promoter-enhancer element, the promoter-enhancer elements are located within or adjacent to the LTR sequences. Preferably, the regulatory sequence is one which is not endogenous to the lentivirus from which the vector is being constructed. Thus, if the vector is being made from SIV, the SIV regulatory sequence found in the SIV LTR would be replaced by a regulatory element which does not originate from SIV.

Marker genes may be utilized to assay for the presence of the vector, and thus, to confirm transfection and integration. The presence of a marker gene ensures the selection and growth of only those host cells that express the inserts. Typical selection genes encode proteins that confer resistance to antibiotics and other toxic substances (e.g., histidinol, puromycin, hygromycin, neomycin, methotrexate). Markers of transduction could also be YFP, RFP, CFP and other intrinsically fluorescent proteins. In another embodiment, cell surface proteins such as clusters of differentiation could be used as markers of transduction.

The vectors are introduced via transfection into the packaging cell line. The packaging cell line produces viral particles that contain the vector genome. Methods for transfection are well known by those of skill in the art. After co-transfection of the packaging vectors and the transfer vector to the packaging cell line, the lentiviral vector is recovered from the culture media and titrated by standard methods used by those of skill in the art. Thus, the packaging constructs can be introduced into human cell lines by transfection using calcium phosphate, lipofection or electroporation.

The packaging vectors could be introduced into cells to generate stable cell lines, generally together with a dominant selectable marker, such as neomycin, DHFR, Glutamine synthetase or ADA (adenosine deaminase), followed by selection in the presence of the appropriate drug and isolation of resistant clones. The selectable marker gene can be linked physically to the packaging genes in the construct. Stable cell lines wherein the packaging functions are configured to be expressed by a suitable packaging cell are known. For example, see U.S. Pat. NOs. 5,686,279; and Ory et al. (Proc. Natl. Acad. Sci., 93, pages 11400-11406, (1996)) that describe packaging cells for MoMuLV-derived vector. The packaging cells with a lentiviral vector incorporated in their genome constitute producer cells. Producer cells are thus cells or cell-lines that can produce or release packaged infectious viral particles carrying the therapeutic gene of interest. These cells can further be adherence-dependent which means that these cells will grow, survive, or maintain function optimally when attached to a surface such as glass or plastic. However, it has been difficult to produce clinical-scale HIV-1-based lentiviral vectors using a packaging cell line, in part due to the toxicity of packaging genes, and gene silencing that occurs during the long culture period necessary for sequential addition of packaging constructs. However, recently the first packaging cell line for prolonged large-scale production of high-titre HIV-1-based lentiviral vector has been described³³.

To obtain virions, the vectors are introduced by transient co-transfection of the packaging cells with one or several helper plasmids, to express proteins complementing the effect of the sequences deleted from the viral genome. For example, for HIV-1 derived vectors, the 293T or HT1080 cell line is transfected simultaneously with the plasmid vector, the plasmid carrying the sequence of VSV-G and the plasmid carrying the sequences coding for gag, pol, tat and rev³⁴. Moreover, the packaging sequences could be delivered into packaging cells by separate plasmid expressing gag, pol, tat and rev respectively³⁵. In the production of minimal vector systems, the producer cell is engineered (either by modification of the viral genome or by the use of helper virus or cosmid) to complement the functions of the parent virus enabling replication and packaging into virions in the producer cell line.

Lentiviral transfer vectors (Naldini et al. "In vivo gene delivery and stable transduction of nondividing cells by a lentiviral vector," Science, 272:263-267, 1996a.; "Efficient transfer, integration, and sustained long-term expression of the transgene in adult rat brains injected with a lentiviral vector," Proc. Natl. Acad. Sci. USA, 93:11382-11388, 1996b) have been used to infect human cells growth-arrested *in vitro* and to transduce neurons after direct injection into the brain of adult rats, hereby demonstrating their usefulness in therapeutic treatments.

B. The SIN Design

The viral vectors of the present invention are self-inactivating recombinant vectors. To meet important human safety needs, the more preferred vectors in accordance with the present invention will not include any other active lentiviral genes, such as gag, pol, env, vpr, vif, vpu, nef, tat, and rev where these genes have been removed or otherwise inactivated. In fact, it is preferred that the only lentiviral sequences present in the vector will be the LTRs, the psi (ψ)-encapsidation sequence, the cPPT, CTS and the RRE. In order to produce lentiviral vector particles containing the lentiviral vector nucleic acids of the present invention in suitable cells, it is sufficient to provide the gag, pol, and rev genes, with or without tat, on a different vector plasmid and to transfect said cells with the lentiviral vector of the present invention and said additional vector plasmid carrying the gag, pol, tat and rev genes and also a vesicular stomatitis virus envelope expression plasmid. Additionally, the gag, pol, tat and rev genes could be loaded on more than one plasmid, in order to maximize the RCR prevention.

The SIN design increases the biosaftey of the lentiviral vectors. The majority of the HIV LTR is comprised of the U3 sequences. The U3 region contains the enhancer and promoter elements that modulate basal and induced expression of the HIV genome in infected cells and in response to cell activation. Several of these promoter elements are essential for viral replication. Some of the enhancer elements are highly conserved among viral isolates and have been implicated as critical virulence factors in viral pathogenesis. The enhancer elements may act to influence replication rates in the different cellular targets of the virus. It is particularly desirable to employ, in the lentiviral vectors of the present invention, a LTR region that has reduced promoter activity relative to wild-type LTR, in that such constructs provide a "self-inactivating" (SIN) biosafety feature. Self-inactivating vectors are ones in which the production of full-length vector RNA in transduced cells is greatly reduced or abolished altogether. This feature minimizes the risk that replication-competent recombinants (RCRs) will emerge. Furthermore, it reduces the risk that cellular coding sequences located adjacent to the vector integration site will be aberrantly expressed. Furthermore, a SIN design reduces the possibility of interference between the LTR and the promoter that is driving the expression of the transgene. It is therefore particularly suitable to reveal the full potential of the internal promoter.

Self-inactivation is preferably achieved through the introduction of a deletion in the U3 region of the 3' LTR of the vector DNA, *i.e.,* the DNA used to produce the vector RNA. Thus, during reverse transcription, this deletion is transferred to the 5' LTR of the proviral DNA. It is desirable to eliminate enough of the U3 sequence to greatly diminish or abolish altogether the transcriptional activity of the LTR, thereby greatly diminishing or abolishing the production of full-length vector RNA in transduced cells. However, it is generally desirable to retain those elements of the LTR that are involved in polyadenylation of the viral RNA, a function spread out over U3, R and U5.

Accordingly, it is desirable to eliminate as many of the transcriptionally important motifs from the LTR as possible while sparing the polyadenylation determinants. In the case of HIV-based lentiviral vectors, it has been discovered that such vectors tolerate significant U3 deletions, including the removal of the LTR TATA box (*e.g.*, deletions from -418 to -18), without significant reductions in vector titres. These deletions render the LTR region substantially transcriptionally inactive in that the transcriptional ability of the LTR is reduced by about 90% or lower. In preferred embodiments the LTR transcription is reduced by about 95% to 99%. Thus, the LTR may be rendered about 90%, 91%, 92%, 93%, 94%, 95%, 96%, 97%, 98%, to about 99% transcriptionally inactive.

As viral transcription starts at the 3' end of the U3 region of the 5' LTR, those sequences are not part of the viral mRNA and a copy thereof from the 3' LTR acts as template for the generation of both LTR in the integrated provirus. If the 3' copy of the U3 region is altered in a retroviral vector construct, the vector RNA is still produced from the intact 5' LTR in producer cells, but cannot be regenerated in target cells. Transduction of such a vector results in the inactivation of both LTR in the progeny virus. Thus, the retrovirus is self-inactivating (SIN) and those vectors are known as SIN transfer vectors.

The SIN design is described in further detail in Zufferey et al. ("Self-inactivating lentivirus vector for safe and efficient in vivo gene delivery," J. Virol., 72, pages 9873-9880, (1998)) and U.S. Pat. No. 5,994,136. As described therein, there are, however, limits to the extent of the deletion at the 3' LTR. First, the 5' end of the U3 region serves another essential function in vector transfer, being required for integration (terminal dinucleotide + att sequence). Thus, the terminal dinucleotide and the att sequence may represent the 5' boundary of the U3 sequences which can be deleted. In addition, some loosely defined regions may influence the activity of the downstream polyadenylation site in the R region. Excessive deletion of U3 sequence from the 3' LTR may decrease polyadenylation of vector transcripts with adverse consequences on both the titers of the vector produce and the transgene expression in target cells. On the other hand, limited deletions may not abrogate the transcriptional activity of the LTR in transduced cells.

The lentiviral vectors described herein carry deletions of the U3 region of the 3' LTR spanning from nucleotide -418 to -18. This is the most extensive deletion and extends as far as the TATA box, therefore abrogating any transcriptional activity of the LTR in transduced cells. The titer of vector in producer cells as well as transgene expression in target cells was unaffected in these vectors. This design therefore provides an enormous increase in vector safety.

SIN-type vectors with such extensive deletions of the U3 region cannot be generated from murine leukaemia virus (MLV) or spleen necrosis virus (SNV) based retroviral vectors without compromising efficiency of transduction.

Elimination of the -418 to -18 nucleotide sequence abolishes transcriptional activity of the LTR, thereby abolishing the production of full length vector RNA in transduced cells. In the HIV-derived lentiviral vectors none of the *in vitro* or *in vivo* properties were compromised by the SIN design.

C. Means of Administration

According to the invention, a vector is introduced into a host cell in need of gene therapy for viral infection. The means of introduction comprises contacting a host capable of being infected with a virus with a vector according to the invention. Preferably, such contacting comprises any means by which the vector is introduced into a host cell; the method is not dependent on any particular means of introduction and is not to be so construed. Means of introduction are well-known to those skilled in the art, and also are exemplified herein. Accordingly, introduction can be effected, for instance, either *in vitro* (*e.g.,* in an *ex vivo* type method of gene therapy) or *in vivo,* which includes the use of electroporation, transformation, transduction, conjugation or triparental mating, transfection, infection, membrane fusion with cationic lipids, high-velocity bombardment with DNA-coated microprojectiles, incubation with calcium phosphate-DNA precipitate, direct microinjection into single cells, and the like. Other methods also are available and are known to those skilled in the art.

The form of the vector introduced into a host cell can vary, depending in part on whether the vector is being introduced *in vitro* or *in vivo.* For instance, the nucleic acid can be closed circular, nicked, or linearized, depending on whether the vector is to be maintained extragenomically (*i.e.,* as an autonomously replicating vector), integrated as a provirus or prophage, transiently transfected, transiently infected as with use of a replication-deficient or conditionally replicating virus, or stably introduced into the host genome through double or single crossover recombination events. Prior to introduction into a host, a vector of the present invention can be formulated into various compositions for use in therapeutic and prophvlactic treatment methods. In particular, the vector can be made into a pharmaceutical composition by combination with appropriate pharmaceutically acceptable carriers or diluents, and can be formulated to be appropriate for either human or veterinary applications.

Thus, a composition for use in the method of the present invention can comprise one or more of the aforementioned vectors, preferably in combination with a pharmaceutically acceptable carrier. Pharmaceutically acceptable carriers are well-known to those skilled in the art, as are suitable methods of administration. The choice of carrier will be determined, in part, by the particular vector, as well as by the particular method used to administer the composition. One skilled in the art will also appreciate that various routes of administering a composition are available, and, although more than one route can be used for administration, a particular route can provide a more immediate and more effective reaction than another route. Accordingly, there are a wide variety of suitable formulations of the composition of the present invention.

A composition comprised of a vector of the present invention, alone or in combination with other antiviral compounds, can be made into a formulation suitable for parenteral administration, preferably intraperitoneal administration. Such a formulation can include aqueous and nonaqueous, isotonic sterile injection solutions, which can contain antioxidants, buffers, bacteriostats, and solutes that render the formulation isotonic with the blood of the intended recipient, and aqueous and nonaqueous sterile suspensions that can include suspending agents, solubilizers, thickening agents, stabilizers, and preservatives. The formulations can be presented in unit dose or multidose sealed containers, such as ampules and vials, and can be stored in a freeze-dried (lyophilized) condition requiring only the addition of the sterile liquid carrier, for example, water, for injections, immediately prior to use. Extemporaneously injectable solutions and suspensions can be prepared from sterile powders, granules, and tablets, as described herein.

An aerosol formulation suitable for administration via inhalation also can be made. The aerosol formulation can be placed into a pressurized acceptable propellant, such as dichlorodifluoromethane, propane, nitrogen, and the like.

The dose administered to an animal, particularly a human, in the context of the present invention should be sufficient to induce a therapeutic response in the infected individual over a reasonable time frame. The dose will be determined by the potency of the particular vector employed for treatment, the severity of the disease state, as well as the body weight and age of the infected individual. The size of the dose also will be determined by the existence of any adverse side effects that can accompany the use of the particular vector employed. It is always desirable, whenever possible, to keep adverse side effects to a minimum.

The dosage can be in unit dosage form, such as a tablet or capsule. The term "unit dosage form" as used herein refers to physically discrete units suitable as unitary dosages for human and animal subjects, each unit containing a predetermined quantity of a vector, alone or in combination with other antiviral agents, calculated in an amount sufficient to produce the desired effect in association with a pharmaceutically acceptable diluent, carrier, or vehicle. The specifications for the unit dosage forms of the present invention depend on the particular compound or compounds employed and the effect to be achieved, as well as the pharmacodynamics associated with each compound in the host. The dose administered should be an "antiviral effective amount" or an amount necessary to achieve an "effective level" in the individual patient.

Since the "effective level" is used as the preferred endpoint for dosing, the actual dose and schedule can vary, depending on interindividual differences in pharmacokinetics, drug distribution, and metabolism. The "effective level" can be defined, for example, as the blood or tissue level desired in the patient that corresponds to a concentration of one or more vector(s) according to the invention, which inhibits a virus, such as HIV, in an assay predictive for clinical antiviral activity of chemical compounds. The "effective level" for compounds of the present invention also can vary when the compositions of the present invention are used in combination with zidovudine or other known antiviral compounds or combinations thereof.

One skilled in the art can easily determine the appropriate dose, schedule, and method of administration for the exact formulation of the composition being used, in order to achieve the desired "effective level" in the individual patient. One skilled in the art also can readily determine and use an appropriate indicator of the "effective level" of the compounds of the present invention by a direct (e.g., analytical chemical analysis) or indirect (e.g., with surrogate indicators of viral infection, such as p24 or reverse transcriptase for treatment of AIDS or AIDS-like disease) analysis of appropriate patient samples (e.g., blood and/or tissues).

Further, with respect to determining the effective level in a patient for treatment of AIDS or AIDS-like disease, in particular, suitable animal models are available and have been widely implemented for evaluating the in vivo efficacy against HIV of various gene therapy protocols (Sarver et al. (1993), AIDS Res. And Hum. Retrovir., 9, 483-487). These models include mice, monkeys and cats. Even though these animals are not naturally susceptible to HIV disease, chimeric mice models (*e.g.,* SCID, bg/nu/xid, bone marrow-ablated BALB/c) reconstituted with human peripheral blood mononuclear cells (PBMCs), lymph nodes, or fetal liver/thymus tissues can be infected with HIV, and employed as models for HIV pathogenesis and gene therapy. Similarly, the simian immune deficiency virus (SIV)/monkey model can be employed, as can the feline immune deficiency virus (FIV)/cat model.

In the treatment of some virally infected individuals, it can be desirable to utilize a "mega-dosing" regimen, wherein a large dose of a vector is administered, time is allowed for the compound to act, and then a suitable reagent is administered to the individual to inactivate the active compound(s). In the method of the present invention, the treatment (*i.e*., the replication of the vector in competition with the virus being treated) is necessarily limited. In other words, as the level, for instance, of HIV decreases, the level of vector dependent on HIV for production of virions will also decrease.

The pharmaceutical composition can contain other pharmaceuticals, in conjunction with a vector according to the invention, when used to therapeutically treat AIDS. These other pharmaceuticals can be used in their traditional fashion (*i*.*e*., as agents to treat HIV infection). In particular, it is contemplated that an antiretroviral agent be employed, such as, preferably, zidovudine. Further representative examples of these additional pharmaceuticals that can be used in addition to those previously described, include antiviral compounds, immunomodulators, immunostimulants, antibiotics, and other agents and treatment regimes (including those recognized as alternative medicine) that can be employed to treat AIDS. Antiviral compounds include, but are not limited to, ddI, ddC, gancylclovir, fluorinated dideoxynucleotides, nonnucleoside analog compounds such as nevirapine (Shih et al., PNAS, 88, 9878-9882 (1991)), TIBO derivatives such as R82913 (White et al., Antiviral Research, 16, 257-266 (1991)), and BI-RJ-70 (Shih et al., Am. J. Med., 90 (Suppl. 4A), 8S-17S (1991)). Immunomodulators and immunostimulants include, but are not limited to, various interleukins, CD4, cytokines, antibody preparations, blood transfusions, and cell transfusions. Antibiotics include, but are not limited to, antifungal agents, antibacterial agents, and anti-Pneumocystis carinii agents.

**Sequence listings**

The SEQ ID NO : 1 of the sequence listings shows the lentiviral vector of the present invention having a 5'LTR from residues 3707 to 3976, wherein the deleted U3 is from 3707 to 3779, the R element is from 3779 to 3871, and U5 is from 3872 to 3976 . Further the promoter is dH2-kappaB promoter from 2218 to 2885. The cPPT/CTS sequence is from 2024 to 2213. Further, the beta-interferon gene is from 2938 to 3498 and is under the control of dH2-kappaB promoter from 2218 to 2885. The 3'LTR is from 1 to 633, wherein U3 is from 1-453, R is from 453-549 and U5 is from 550 to 633.

**Figure legends**

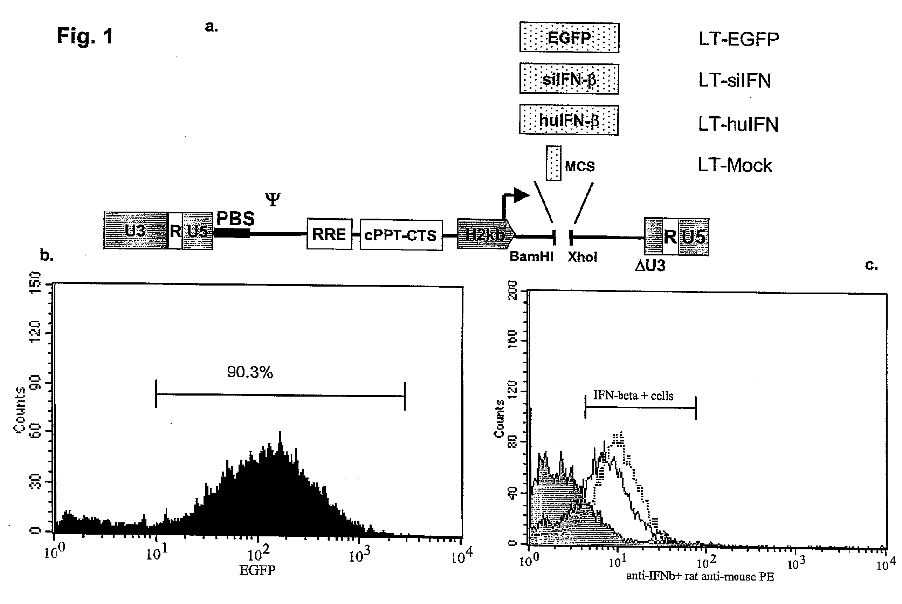

**Figure 1** a. shows a schematic representation of the constructs used in these studies. Particularly, the figure shows the structure of the lentiviral vector (LT) encoding EGFP, si-IFNβ, hu-IFNβ and a multi-cloning site (MCS) under the control of the H2-kb promoter. Batches of the four different vectors were produced simultaneously to minimize batch variations. **b**. FACS analysis of EGFP expression of CEMx174 cells with LT-EGFP, percentage of GFP⁺ cells is indicated in the dot-plot **c.** Endogenous production of IFN-β in transduced cells. Cells were permeabilized, labeled with a mAB specific for huIFN-β (clone 8.PL.1) and analyzed by FACS for the presence of IFN-β. Full area, dotted line, and full line represent IFN-β expression in LT-Mock, LT-siIFN and LT-huIFN transduced cells, respectively.

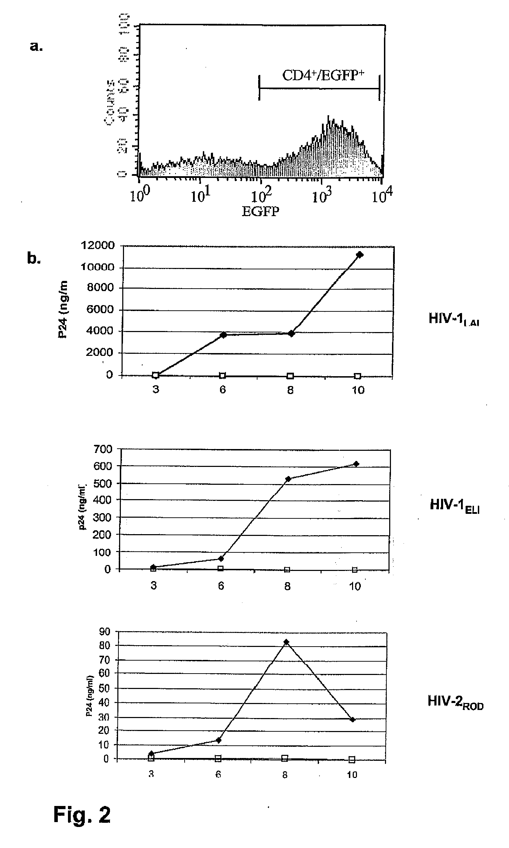

**Figure 2** shows effective inhibition of different strains of HIV in LT-huIFN transduced cells. One representative experiment is shown. CD4⁺ cells were transduced in parallel with the vectors, washed twice and cultured for 3 days. Transduced cells were infected overnight with 10ng P24/ml of different strains of HIV (LAI, ELI and ROD), washed twice and cultured at 1.5x10⁶ cells/ml. Cell counts were normalized after each passage and culture supernatants were harvested for viral loads analysis. **a**, Efficiency of EGFP expression in CD4⁺ cells transduced with LT-EGFP was measured by FACS analysis 3 days after the end of transduction. **b**. Evolution of viremia during post-infection culture was represented as ng of P24/ml at different days of culture. Used virus strains are listed next to each specific graph. LT-Mock transduced cells are represented by full squares and LT-huIFN transduced cells are represented by open squares, respectively.

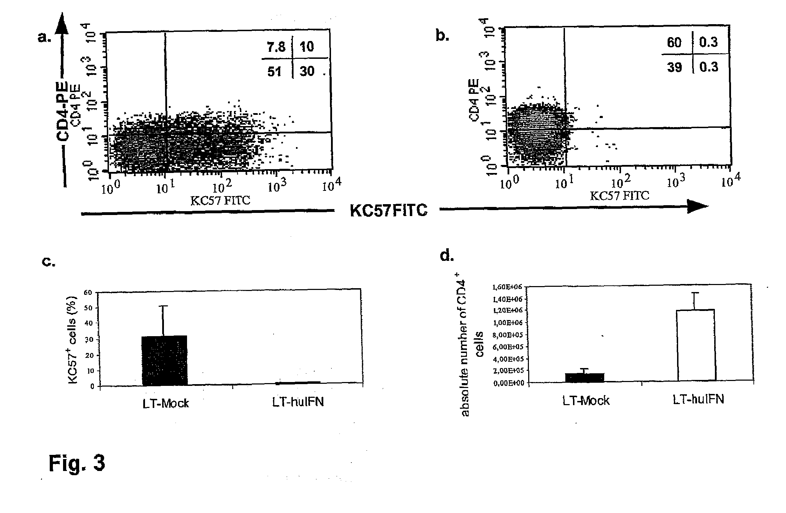

**Figure 3** shows that the expression of huIFN-β inhibits infection by HIV and preserves CD4⁺ cells. Transduced CD4⁺ cells were infected by 10ng/ml of HIV-1_{LAI}. The presence of intracellular HIV and expression of CD4 were tested 5 days post-infection. Cells were harvested, counted, permeabilized and labeled with KC57-FITC and CD4-PE. **a and b**. FACS analysis of one representative experiment is presented for LT-Mock and LT-huIFN transduced cells, respectively. **c**. Percentage of KC57⁺ cells at 5 days post-infection. Shown are the means ± SD. **d**. Absolute number of CD4⁺ cells generated in the culture. Shown are the averages ± SD.

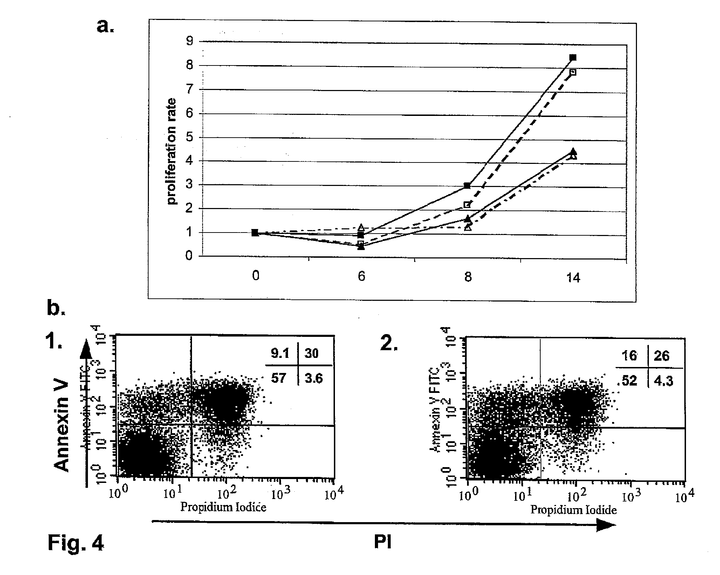

**Figure 4** shows that the exogenous huIFN-β expression does not affect proliferation and apoptosis in CD4⁺ cells. CD4⁺ cells purified from PBMC of healthy donors were transduced with 1250ng p24/ml of the vectors, this dose corresponds to 0,7 TU/ml or 2.13±1.07 of MOI. Cells were numerated and replated at 1.10⁶ cells/ml at day 6, 8 and 10 of culture. **1**, Proliferation of CD4⁺ cells is expressed as rate of cells in the culture comparing to day 0. Two representative experiments are shown. Results from experiment 1 are represented by full and open squares; those from experiment 2 by full and open triangles, wherein dotted lines represent the LT-Mock transduced cells whereas full lines represent LT-hulFN ones. **2,** Study of apoptosis is performed after staining with Annexin V and propidium iodide (PI) at day 10 of culture. FACS Analysis of one representative experiment is shown here. **a and b,** analysis in LT-Mock and LT-huIFN transduced cells, respectively. Percentages of cells in every quadrant are indicated within the dot-plots.

**Examples**

**1. Cell lines:**

The CEMx174 human lymphoid cells, highly permissive for replication of various HIV/SIV strains³⁶, are cultured in RPMI complete medium (RPMI 1640 supplemented with 10% heat-inactivated foetal calf serum, 2mM glutamine, 100 U/ml of penicillin, and 100 µg/ml of streptomycin).

293FT cells (Invitrogen) grown in DMEM medium supplemented with 10% foetal calf serum, 2mM glutamine, 100 U/ml of penicillin, and 100 µg/ml of streptomycin, 0.1 mM MEM Non-Essential Amino Acids and 500 µg/ml of G418.

**2. Production of HIV-1-derived vectors**

Vectors particles were produced by transient calcium phosphate co-transfection of 293 FT cells with the vector plasmid carrying the human or simian IFN-β or EGFP, pCMVΔ8.91 and pHCMV-G as previously described³⁴. Vectors concentrations were determined by measuring p24 (HIV-1 capsid protein). The viral titres were evaluated on the CEMx174 cells and expressed in TU/ml (Transduction Unit/ml). The batches of the different vectors used were prepared together to ensure similar transduction efficiencies³⁷.

**3. Production of virus strains**

HIV-1 (strains X4 LAI and ELI) and HIV-2 (strain A ROD) were obtained from the NIBSC (National Institute for Biological Standards and Control, www.nibsc.ac.uk), SIVmac (strains 251 and 239) were obtained from the Pasteur Institute (Paris, France). HIV and SIV strains were amplified on CEMx174. Cell supernatants were centrifuged to eliminate the debris and viral stocks were titrated by determination of the SIV or HIV gag antigenemia by ELISA p27 (Coulter) or ELISA p24 (Innogenetics) respectively and stored at -80°C.

**4. Isolation of primary cells**

Buffy coat was obtained from the blood transfusion centre (CHU, Liège, Belgium). CD4⁺ cells were purified from PBMC (Peripheral Blood Mononuclear Cells) using the CD4 Dynabeads (Dynal) according to the manufacturer's instructions.

**5. Flow cytometry:**

Surface staining was performed with CD4-PE Mab (Monoclonal Antibody, clone L200, Becton Dickinson). Intracellular staining of p24 protein was performed with a specific antibody (KC57-FITC Mab, Coulter), using a cell permeabilization kit according to manufacturer's recommendations (Oxford Biotechnology). Phenotype analyses were realized by Flow cytometry (FACSvantage, Becton Dickinson). Apoptosis was determined using the Annexin-V FITC kit (Becton Dickinson) following the manufacturer's instructions.

**6. Transduction protocols**

CEMx174 cells were plated at 5x10⁵cells/ml. Lentiviral particles were added (500 ng/ml of viral p24). After 12 h of transduction, cells were washed and cultured in the same conditions during 72 h. EGFP expression in the cells transduced with the LT-EGFP vector was determined by flow cytometry analysis.

Human PBMC or CD4⁺ cells were plated at 10⁶ cells/ml in X-Vivo 15 medium (Cambrex) complemented with 10% huSAB (human Serum AB, Cambrex), 50 IU/ml rHu-IL-2 (Gentaur) and CD3-CD28 Dynabeads (3 beads:1 cell-ratio, Dynal) in plates coated with retronectin (25 µg/ml, Takara). Vector particles were added at the concentration of 1250 ng of viral p24/ml, twice at a 24-hour interval for a total of 48 h. Cells were then washed and cultured in conditions of T-cell expansion. EGFP expression in the CD4⁺ cell population was analysed by flow cytometry after 72 h of culture.

**7. Construction of LT-siIFN-β, L-Mock and LT-EGFP vectors**

The BamHI-EcoRI fragment of the pUC0.6-siIFN-β was subcloned in pBluescript KS-II (Stratagene), generating the plasmid pBKS-II-siIFN. The LT-silFNβ plasmid was obtained by replacing the BamHI-Xhol fragment, containing the human IFN-β cDNA of LT-huIFNβ, by the BamHI-Xhol fragment of pBKS-II-silFN. LT-EGFP was obtained by cloning the BamHI-Xhol fragment of the TRIP-ΔU3-EF1α-EGFP plasmid in LT-siIFN-β. LT-Mock was obtained by substitution of the Sacl-Kpnl fragment of LT-EGFP containing the EGFP with the 102 bp Sacl-Kpnl fragment of the pBluescript KS-II multicloning site (Figure 1,a.). To evaluate the feasibility of our model, we have transduced CEMx174 cells with the different vectors. FACS analysis of LT-EGFP transduced cells was performed 72 hours after the end of transduction (Figure 1, b.) and showed a high proportion of EGFP-expressing CEMx174, close to 95 ± 3% (n=4). Therefore, we chose this time point to collect cells and supernatants from control and IFN-β transduced cultures. Flow cytometric analyses of intra-cytoplasmic IFN-β (Figure 1, c.) showed that cells transduced with LT-huIFN and LT-siIFN produce huIFNβ and siIFNβ, respectively, contrary to cells transduced with LT-Mock. ELISA analysis of supernatants of cells transduced with LT-huIFN indicated the presence of IFN-β at a level of 560 ± 77 IU/ml (International Unit / ml) (n=3), with no detectable IFN-β in the supernatant of cells transduced with control vectors.

**8. Inhibition of HIV replication in LT-IFN transduced primary cells**

To evaluate the protective effects of huIFN-β expression during T cell infection by HIV, CD4⁺ cells purified from PBMC of healthy donors were transduced with the different LT vectors using the transduction protocol described above. Transduction efficiency with the LT-EGFP vector determined 72 hours later was 86±10% of CD4⁺ cells (n=4) (Figure 2,a.). We next examined the antiviral resistance of huIFN-β-expressing lymphocytes. Transduced cells challenged with several common strains of HIV were cultured for 10 days. The measure of virion production in culture supernatants by p24 ELISA at different times post-challenge during 10 days revealed that, in LT-mock-transduced cells, HIV replication increased rapidly and became maximal after 6-8 days of culture. Conversely, LT-huIFN-transduced cells displayed a high anti-HIV resistance, as illustrated by the 99,8±0,1, 99±1 and 99,2±0.5% reduction of p24 release in the supernatants of CD4+ cells infected with HIV-1 _{LAI}, HIV-1 _{ELI} and HIV-2 _{ROD} respectively (p<0.005) (Figure 2,b; Table 1). Interestingly, the same range of inhibition was obtained with IFN-β-transduced PBMC challenged with the same strains of virus. Percentages of inhibition were 99.2±1.1, 99±1 and 95±9% in cells infected with LAI (n=6), ELI (n=4) and ROD (n=3), respectively (p<0.005). These results clearly indicated that exogenous expression of huIFN-β drastically inhibits the replication of different HIV strains.

**9. Expression of exogenous huIFN-β leads to defects in the assembly of HIV-1 _{LAI} and blocks CD4 down-regulation:**

Here we have studied CD4 expression by infected cells and the formation of virions by transduced cells. Transduced cells were subsequently challenged with HIV-1_{LAI} as previously described, infected cells were counted at different times of culture and expression of CD4 and intra-cellular p24 was assayed at day 10. Results indicated that expression of huIFN-β preserves CD4 expression at the surface of infected cells compared with LT-Mock transduced cells (Figures 3), so CD4⁺ cells represented 15±5% of infected cells previously transduce with the Mock vector, compared to 51±14% in huIFN-β expressing cells. Cell proliferation was different during this study, x1.2±0.3 and x3.5±1.3 for LT-Mock- and LT-huIFN-transduced cells respectively (n=3, p<0.05), so the absolute number of CD4⁺ cells is greatly improved (x 11±6) in hu-IFNβ-expressing cells in contrast with cells transduced with the control vector. Moreover, expression of hu-IFNβ drastically alters the formation of the p24 subunit of HIV gag protein (Figure 3, c.), so that the proportion of KC57⁺ cells was decreased from 31±20% in control cells (n=3, p=0.053) to 0.4±0.3% in cells transduced with huIFN-β. These results suggested that the expression of huIFN-β blocks the CD4 down-regulation and interfered with the steps of the viral cycle leading to the expression of the HIV gag protein.

**10. Production of exogen huIFN-β has no negative effect on human T lymphocytes**

Production of huIFN-β was monitored in the supernatants after 8 days of culture and LT-Mock transduced cells did not produce detectable levels of huIFN-β, contrary to LT-huIFN transduced cells that produced 14±5 IU/10⁵ cells per 3 days (n=4). In these experiments, huIFN-β expression did not alter the proliferation rate as compared with cells transduced with the Mock vector (Figure 4, a). Furthermore, such level of huIFN-β expression has no effect on cell apoptosis (Figure 4, b; Table 2).

**11. Inhibition of HIV and SIV replication on IFN-β transduced CEMx174**

Moreover, we have analyzed the HIV inhibition by CEMx174 cell transduced with the different vectors. We then challenged transduced cells with different strains of HIV and SIV at a concentration of 10 ng of p24 or p27/ml. Viral replication in the culture supernatants was measured at different times by ELISA dosing the p24 or p27 proteins, for HIV and SIV strains respectively. Table 3 showed that IFN-β expression in CEMx174 cells drastically reduced HIV and SIV replication. The amounts of HIV-1 _{LAI}, HIV-1 _{ELI} and HIV-2 _{ROD} released in the culture supernatant was reduced by a factor ranging between 97±7%, 99.9±0.1% and 99.8±0.1% respectively when CEMx174 cells were transduced with LT-huIFN compared to cells transduced with LT-Mock vector. Similar results were observed when IFN-β-transduced cells were infected with pathogenic SIV strains, with an inhibition of 99.3±0.3% for SIVmac-239 and of 98.8±1.3% for SIVmac-251. The level of inhibition observed was identical when LT-silFN was used (Table 3). These results demonstrated that expression of exogenous huIFN-β and siIFN-β conferred similar antiviral protection against several HIV and SIV pathogenic strains.

**Table 1. Exogenous IFN-β inhibits HIV replication in LT-IFN transduced cells. Transduced cells were challenged with different strains of HIV 72 hours after the end of transduction. Viral load was determined at different times by ELISA p24 on culture supernatants. Inhibition level is expressed in average of percentages ± SD (p, T student test was performed to establish whether the inhibition rate observed is significant).**

| **cells** | | ***HIV-1 LAI*** | ***HIV-1 ELI*** | ***HIV-2 ROD*** |
|---|---|---|---|---|
| **CD⁴⁺ cells** | % of inhibition *(p)* | 99.9±0.11 | 99±1 | 99.26±0.6 |
| | | *(<0.005) n*=*9* | *(<0.005) n*=*7* | *(<0.005) n*=*3* |
| **PBMC** | | 99.26±1.14 | 99.08±1.1 | 94.94±8.8 |
| | | *(<0.005) n*=*6* | *(<0.005) n*=4 | *(<0.005) n*=4 |

**Table 2. huIFN-β expression does not affect T cell expansion and survival (n=4). a. Detection of huIFN-β in culture supernatant of transduced cells was performed 8 days after transduction. Amount of huIFN-β produced by transduced cells is expressed in IU (International Unit)/10⁵ transduced cells ± SD. b. Influence of exogenous huIFN-β on apoptosis was studied 8 days post-transduction. Proportion of AnnexinV⁺ cells is notified in percentage ± SD. c. Proliferation rate of transduced cells is expressed by the fold of expansion after 10 days of culture.**

| **construct** | **Transduction efficiency (with LT-EGFP)** | **IFN-β (UI/10⁵ cell) (a)** | **Annexin V⁺ cells (%) (b)** | **Proliferation rate (c)** |
|---|---|---|---|---|
| **LT-Mock** | 86,122 ± 10,06 | <0,001 | 50,95 ± 12,31 | 8,08 ± 3,37 |
| **LT-huIFN** | | 14,34 ± 5,35 | 49,9 ± 10,93 | 8,63 ± 4,02 |

**Table 3: Inhibition of different strains of HIV/SIV in LT-IFN CEMx174 transduced cells. Inhibition level is expressed as an average of percentage ± SD (p), T student test was performed to determined whether the inhibition rate observed is significant. *Values of p superior to 0.05 could be explained by the low number of experiments (n=3).**

| **construct** | ***HIV-1 _{LAI} (n=3)*** | ***HIV-1 _{ELI} (n=3)*** | ***HIV-2 _{ROD} (n=5)*** | ***SIVmac239 (n=6)*** | ***SIVmac251 (n=6)*** |
|---|---|---|---|---|---|
| **LT-huIFN** | **97.0±7** | **99.96±0.05** | **99.83±0.12** | **99.35±0.27** | **98.84±1.33** |
| | (0.092*) | (<0.05) | (<0.05) | (<0.05) | (<0.05) |
| **LT-siIFN** | **97.4±4** | **99.98±0.02** | **99.23±0.48** | **99.68±0.15** | **98.2±1.25** |
| | (0.090*) | (<0.05) | (0.089*) | (<0.05) | (<0.05) |

### References

1. Shafer RW, Vuitton DA. Highly active antiretroviral therapy (HAART) for the treatment of infection with human immunodeficiency virus type 1. Biomed Pharmacother. 1999;53:73-86.
2. Siliciano JD, Kajdas J, Finzi D, et al. Long-term follow-up studies confirm the stability of the latent reservoir for HIV-1 in resting CD4+ T cells. Nat Med. 2003;9:727-728.
3. Nunez M, Soriano V. Hepatotoxicity of antiretrovirals: incidence, mechanisms and management. Drug Saf. 2005;28:53-66.
4. Nolan D, Reiss P, Mallal S. Adverse effects of antiretroviral therapy for HIV infection: a review of selected topics. Expert Opin Drug Saf. 2005;4:201-218.
5. Turner BJ. Adherence to antiretroviral therapy by human immunodeficiency virus-infected patients. J Infect Dis. 2002;185 Suppl 2:S143-151.
6. Tang JW, Pillay D. Transmission of HIV-1 drug resistance. J Clin Virol. 2004;30:1-10.
7. Pillay D, Taylor S, Richman DD. Incidence and impact of resistance against approved antiretroviral drugs. Rev Med Virol. 2000;10:231-253.
8. Chen JD, Bai X, Yang AG, Cong Y, Chen SY. Inactivation of HIV-1 chemokine co-receptor CXCR-4 by a novel intrakine strategy. Nat Med. 1997;3:1110-1116.
9. Chinen J, Aguilar-Cordova E, Ng-Tang D, Lewis DE, Belmont JW. Protection of primary human T cells from HIV infection by Trev: a transdominant fusion gene. Hum Gene Ther. 1997;8:861-868.
10. Chang HK, Gendelman R, Lisziewicz J, Gallo RC, Ensoli B. Block of HIV-1 infection by a combination of antisense tat RNA and TAR decoys: a strategy for control of HIV-1. Gene Ther. 1994;1:208-216.
11. Chaloin L, Lehmann MJ, Sczakiel G, Restle T. Endogenous expression of a high-affinity pseudoknot RNA aptamer suppresses replication of HIV-1. Nucleic Acids Res. 2002;30:4001-4008.
12. Browning CM, Cagnon L, Good PD, Rossi J, Engelke DR, Markovitz DM. Potent inhibition of human immunodeficiency virus type 1 (HIV-1) gene expression and virus production by an HIV-2 tat activation-response RNA decoy. J Virol. 1999;73:5191-5195.
13. Bai J, Gorantla S, Banda N, Cagnon L, Rossi J, Akkina R. Characterization of anti-CCR5 ribozyme-transduced CD34+ hematopoietic progenitor cells in vitro and in a SCID-hu mouse model in vivo. Mol Ther. 2000;1:244-254.
14. Kim JH, McLinden RJ, Mosca JD, Vahey MT, Greene WC, Redfield RR. Inhibition of HIV replication by sense and antisense rev response elements in HIV-based retroviral vectors. J Acquir Immune Defic Syndr Hum Retrovirol. 1996;12:343-351.
15. Jacque JM, Triques K, Stevenson M. Modulation of HIV-1 replication by RNA interference. Nature. 2002;418:435-438.
16. Qin XF, An DS, Chen IS, Baltimore D. Inhibiting HIV-1 infection in human T cells by lentiviral-mediated delivery of small interfering RNA against CCR5. Proc Natl Acad Sci U S A. 2003;100:183-188.
17. Novina CD, Murray MF, Dykxhoorn DM, et al. siRNA-directed inhibition of HIV-1 infection. Nat Med. 2002;8:681-686.
18. Strayer DS, Akkina R, Bunnell BA, et al. Current status of gene therapy strategies to treat HIV/AIDS. Mol Ther. 2005;11:823-842.
19. Vieillard V, Lauret E, Rousseau V, De Maeyer E. Blocking of retroviral infection at a step prior to reverse transcription in cells transformed to constitutively express interferon beta. Proc Natl Acad Sci U S A. 1994;91:2689-2693.
20. Kornbluth RS, Oh PS, Munis JR, Cleveland PH, Richman DD. The role of interferons in the control of HIV replication in macrophages. Clin Immunol Immunopathol. 1990;54:200-219.
21. Hansen BD, Nara PL, Maheshwari RK, et al. Loss of infectivity by progeny virus from alpha interferon-treated human immunodeficiency virus type 1-infected T cells is associated with defective assembly of envelope gp120. J Virol. 1992;66:7543-7548.
22. Poli G, Orenstein JM, Kinter A, Folks TM, Fauci AS. Interferon-alpha but not AZT suppresses HIV expression in chronically infected cell lines. Science. 1989;244:575-577.
23. Baca-Regen L, Heinzinger N, Stevenson M, Gendelman HE. Alpha interferon-induced antiretroviral activities: restriction of viral nucleic acid synthesis and progeny virion production in human immunodeficiency virus type 1-infected monocytes. J Virol. 1994;68:7559-7565.
24. Shirazi Y, Pitha PM. Interferon alpha-mediated inhibition of human immunodeficiency virus type 1 provirus synthesis in T-cells. Virology. 1993;193:303-312.
25. Coccia EM, Krust B, Hovanessian AG. Specific inhibition of viral protein synthesis in HIV-infected cells in response to interferon treatment. J Biol Chem. 1994;269:23087-23094.
26. Vieillard V, Cremer I, Lauret E, et al. Interferon beta transduction of peripheral blood lymphocytes from HIV-infected donors increases Th1-type cytokine production and improves the proliferative response to recall antigens. Proc Natl Acad Sci U S A. 1997;94:11595-11600.
27. Hadida F, De Maeyer E, Cremer I, et al. Acquired constitutive expression of interferon beta after gene transduction enhances human immunodeficiency virus type 1-specific cytotoxic T lymphocyte activity by a RANTES-dependent mechanism. Hum Gene Ther. 1999;10:1803-1810.
28. Vieillard V, Jouveshomme S, Leflour N, et al. Transfer of human CD4(+) T lymphocytes producing beta interferon in Hu-PBL-SCID mice controls human immunodeficiency virus infection. J Virol. 1999;73:10281-10288.
29. Gay W, Lauret E, Boson B, et al. Low autocrine interferon beta production as a gene therapy approach for AIDS: Infusion of interferon beta-engineered lymphocytes in macaques chronically infected with SIVmac251. Retrovirology. 2004;1:29.
30. Amsellem S, Ravet E, Fichelson S, Pflumio F, Dubart-Kupperschmitt A. Maximal lentivirus-mediated gene transfer and sustained transgene expression in human hematopoietic primitive cells and their progeny. Mol Ther. 2002;6:673-677.
31. Humeau LM, Binder GK, Lu X, et al. Efficient lentiviral vector-mediated control of HIV-1 replication in CD4 lymphocytes from diverse HIV+ infected patients grouped according to CD4 count and viral load. Mol Ther. 2004;9:902-913.
32. Depienne C, Mousnier A, Leh H, et al. Characterization of the nuclear import pathway for HIV-1 integrase. J Biol Chem. 2001;276:18102-18107.
33. Ni Y, Sun S, Oparaocha I, et al. Generation of a packaging cell line for prolonged large-scale production of high-titer HIV-1-based lentiviral vector. J Gene Med. 2005;7:818-834.
34. Sirven A, Pflumio F, Zennou V, et al. The human immunodeficiency virus type-1 central DNA flap is a crucial determinant for lentiviral vector nuclear import and gene transduction of human hematopoietic stem cells. Blood. 2000;96:4103-4110.
35. Pawliuk R, Westerman KA, Fabry ME, et al. Correction of sickle cell disease in transgenic mouse models by gene therapy. Science. 2001;294:2368-2371.
36. Salter RD, Howell DN, Cresswell P. Genes regulating HLA class I antigen expression in T-B lymphoblast hybrids. Immunogenetics. 1985;21:235-246.
37. Ravet E, Reynaud D, Titeux M, et al. Characterization of DNA-binding-dependent and -independent functions of SCL/TAL1 during human erythropoiesis. Blood. 2004;103:3326-3335.

## Claims

1. A self-inactivating recombinant lentiviral vector comprising a 5' LTR and a 3' LTR, wherein the LTR region has been rendered substantially transcriptionally inactive by virtue of deletions in the U3 region of the 3' LTR, **characterized in that** the lentiviral vector is further comprising a beta-interferon gene positioned under the control of a promoter.

2. The lentiviral vector of claim 1, at least comprising the following elements: 5' LTR, PBS (primer binding site), Psi-site, RRE (Rev Responsive Element), cPPT and CTS sequence, the promoter for expression the beta-interferon gene, the beta-interferon gene, and 3' LTR.

3. The lentiviral vector according to claim 1 or 2, **characterized in that** said promoter is active to promote detectable transcription of the beta-interferon gene in human cells presenting a target for human immunodeficiency virus.

4. The lentiviral vector according to claim 3, **characterized in that** the human cells presenting a target for human immunodeficiency virus are selected from the group consisting of CD4⁺ cells, peripheral blood mononuclear cells (PBMC) and macrophages.

5. The lentiviral vector according to any one of claims 1 to 4, **characterized in that** said promoter is active to promote detectable transcription of the beta-interferon gene in cells selected from the group consisting of CD4⁺ cells, PBMC, macrophages, CD34⁺ cells and haematopoietic stem cells.

6. The lentiviral vector according to any one of claims 1 to 5, **characterized in that** the promoter is H2-kappa-B promoter.

7. The lentiviral vector according to any one of claims 1 to 6, **characterized in that** the LTR regions have reduced promoter activity compared to the wild-type LTR.

8. The lentiviral vector according to any one of claims 1 to 7, **characterized in that** the vector is incapable of reconstituting a wild-type lentivirus through recombination.

9. The lentiviral vector according to any one of claims 1 to 9, **characterized in that** said U3 region of 3 'LTR deleted is all of said U3 region except for a 5' terminal dinucleotide and an att sequence.

10. A plasmid, comprising the sequence of the lentiviral vector according to any one of claims 1 to 9.

11. Use of the lentiviral vector according to any one of claims 1 to 10 for transducing cells selected from the group consisting of CD4⁺ cells, monocytes, PBMC, macrophages, CD34⁺ cells and haematopoietic stem cells.

12. A cell having the lentiviral vector according to any one of claims 1 to 10 stably integrated into the chromosomal DNA.

13. The cell according to claim 12, **characterized in that** the cell is selected from the group consisting of CD4⁺ cells, monocytes, PBMC, macrophages, CD34⁺ cells and haematopoietic stem cell.

14. Use of the lentiviral vector according to any one of claims 1 to 10 for the preventive and/or therapeutic treatment of HIV infection.

15. Use of the lentiviral vector according to any one of claims 1 to 10 for the inhibition of the replication of HIV.

16. Medicament comprising the lentiviral vector according to any one of claims 1 to 10.

17. Use of the lentiviral vector according to any one of claims 1 to 10 for the manufacture of a medicament for the preventive and/or therapeutic treatment of HIV infection.

18. Use of the lentiviral vector according to any one of claims 1 to 10 for the manufacture of a medicament for the inhibition of the replication of HIV.

19. Method for the transduction of cells comprising the step of transduction of cells with the vector according to claims 1 to 10, wherein the cells are selected from the group consisting of CD4⁺ cells, monocytes, PBMC, macrophages, CD34⁺ cells and haematopoietic stem cells.

20. Method for the preventive and/or therapeutic treatment of a human individual comprising the following steps:
- transduction of cells with the vector according to claims 1 to 10, wherein the cells are selected from the group consisting of CD4⁺ cells, monocytes, PBMC, macrophages, CD34⁺ cells and haematopoietic stem cells; and
- administration of the transduced cells into the human individual.


SEQUENCE LISTING

<110> Université de Liège

<120> A self-inactivating recombinant lentiviral vector for the inhibition of HIV replication

<130> P21734EP

<160> 1

<170> PatentIn version 3.1

<210> 1
<211> 3976
<212> DNA
<213> artificial sequence

<220>
<223> lentiviral vector
<220>
<221> promoter
<222> (2218)..(2885)
<223> dH2-kappaB promoter

<220>
<221> LTR
<222> (3707)..(3976)
<223> 5´LTR, R: 3779-3871; U5: 3872-3976, dU3: 3707-3779

<220>
<221> LTR
<222> (1)..(633)
<223> 3´LTR; U3: 1-453; R: 453-549; U5 550-633

<220>
<221> misc_feature
<222> (2024)..(2213)
<223> cPPT/CTS

<220>

<221>  gene

<222>  (2938)..(3498)

<223>

<400>  1
ggaagggcta attcactccc aaagaagaca agatatcctt gatctgtgga tctaccacac      60

acaaggctac ttccctgatt agcagaacta cacaccaggg ccaggggtca gatatccact     120

gacctttgga tggtgctaca agctagtacc agttgagcca gataaggtag aagaggccaa     180

taaaggagag aacaccagct tgttacaccc tgtgagcctg catgggatgg atgacccgga     240

gagagaagtg ttagagtgga ggtttgacag ccgcctagca tttcatcacg tggcccgaga     300

gctgcatccg gagtacttca agaactgctg atatcgagct tgctacaagg gactttccgc     360

tggggacttt ccagggaggc gtggcctggg cgggactggg gagtggcgag ccctcagatc     420

ctgcatataa gcagctgctt tttgcctgta ctgggtctct ctggttagac cagatctgag     480

cctgggagct ctctggctaa ctagggaacc cactgcttaa gcctcaataa agcttgcctt     540

gagtgcttca agtagtgtgt gcccgtctgt tgtgtgactc tggtaactag agatccctca     600

gaccctttta gtcagtgtgg aaaatctcta gcagtggcgc ccgaacaggg acttgaaagc     660

gaaagggaaa ccagaggagc tctctcgacg caggactcgg cttgctgaag cgcgcacggc     720

aagaggcgag gggcggcgac tggtgagtac gccaaaaatt ttgactagcg gaggctagaa     780

ggagagagat gggtgcgaga gcgtcagtat taagcggggg agaattagat cgcgatggga     840

aaaaattcgg ttaaggccag ggggaaagaa aaaatataaa ttaaaacata tagtatgggc     900

aagcagggag ctagaacgat tcgcagttaa tcctggcctg ttagaaacat cagaaggctg     960

tagacaaata ctgggacagc tacaaccatc ccttcagaca ggatcagaag aacttagatc    1020

attatataat acagtagcaa ccctctattg tgtgcatcaa aggatagaga taaaagacac    1080

caaggaagct ttagacaaga tagaggaaga gcaaaacaaa agtaagacca ccgcacagca    1140

agcggccgct gatcttcaga cctggaggag gagatatgag ggacaattgg agaagtgaat    1200

tatataaata taaagtagta aaaattgaac cattaggagt agcacccacc aaggcaaaga    1260

gaagagtggt gcagagagaa aaaagagcag tgggaatagg agctttgttc cttgggttct    1320

tgggagcagc aggaagcact atgggcgcag cgtcaatgac gctgacggta caggccagac    1380

aattattgtc tggtatagtg cagcagcaga acaatttgct gagggctatt gaggcgcaac    1440

agcatctgtt gcaactcaca gtctggggca tcaagcagct ccaggcaaga atcctggctg    1500

tggaaagata cctaaaggat caacagctcc tggggatttg gggttgctct ggaaaactca    1560

tttgcaccac tgctgtgcct tggaatgcta gttggagtaa taaatctctg gaacagattt    1620

ggaatcacac gacctggatg gggtgggaca gagaaattaa caattacaca agcttaatac    1680

actccttaat tgaagaatcg caaaaccagc aagaaaagaa tgaacaagaa ttattggaat    1740

tagataaatg ggcaagtttg tggaattggt ttaacataac aaattggctg tggtatataa    1800

aattattcat aatgatagta ggaggcttgg taggtttaag aatagttttt gctgtacttt    1860

ctatagtgaa tagagttagg cagggatatt caccattatc gtttcagacc cacctcccaa    1920

ccccgagggg acccgacagg cccgaaggaa tagaagaaga aggtggagag agagacagag    1980

acagatccat tcgattagtg aacggatctc gacggtatcg ccgaattcac aaatggcagt    2040

attcatccac aattttaaaa gaaaaggggg gattgggggg tacagtgcag gggaaagaat    2100

agtagacata atagcaacag acatacaaac taaagaatta caaaaacaaa ttacaaaaat 2160

tcaaaatttt cgggtttatt acagggacag cagagatcca ctttggctga tacgcgtaag 2220

cttgcagcca cagacttttc atctaggaat taacacaagg aatctgtgtc tcagcacagg 2280

gctgagaaga cagatcctga ggggagaggc aaagtctaca cttaacagat gagagtcctg 2340

cactcaggct tggcagtgtg agccgcccat tgcaggtgaa cagagcctgg tctctgtggg 2400

gtccctgtgg ggcttgcagc ccagcgcctt gactttaagg aaaagcctct ctctccactg 2460

catccctaag cgcttgtgtc gccattgtat tcccggaaga ggcttttctt ctagaagact 2520

ccagggtctg acttctgaag agaagaagaa agaggaagag tggaagagag gacacagaga 2580

gtctggcctg cgggtctctc ctggtgtttt gagagtttct ggatcagaac tcggagacga 2640

cagcacaggg ttcaggcaaa gtcttagtcg ccaggcagtg aggtcagggg tggggaagcc 2700

cagggctggg gattccccat ctccacagtt tcacttctgc acctaacctg ggtcaggtcc 2760

ttctgtccgg acactgttga cgcgcagtca gctcttaccc ccattgggtg gcgcgatcac 2820

caagaaccaa tcagtgtcgc cgcggacgct ggatataaag tccacgcagc ccgcagaact 2880

cagaagtcgt ggtcgactct agaggatccc ctaggcgaca ctgttcgtgt tgtcaacatg 2940

accaacaagt gtctcctcca aattgctctc ctgttgtgct tctccactac agctctttcc 3000

atgagctaca acttgcttgg attcctacaa agaagcagca attttcagtg tcagaagctc 3060

ctgtggcaat tgaatgggag gcttgaatat tgcctcaagg acaggatgaa ctttgacatc 3120

cctgaggaga ttaagcagct gcagcagttc cagaaggagg acgccgcatt gaccatctat 3180

gagatgctcc agaacatctt tgctattttc agacaagatt catctagcac tggctggaat 3240

gagactattg ttgagaacct cctggctaat gtctatcatc agataaacca tctgaagaca 3300

gtcctggaag aaaaactgga gaaagaagat ttcaccaggg gaaaactcat gagcagtctg 3360

cacctgaaaa gatattatgg gaggattctg cattacctga aggccaagga gtacagtcac 3420

tgtgcctgga ccatagtcag agtggaaatc ctaaggaact tttacttcat taacagactt 3480

acaggttacc tccgaaactg aagatctcct agcctgctcg agacctagaa aaacatggag 3540

caatcacaag tagcaataca gcagctacca atgctgattg tgcctggcta gaagcacaag 3600

aggaggagga ggagggtgtt ccagtcacac ctcaggtacc tttaagacca atgacttaca 3660

aggcagctgt agatcttagc cactttttaa aagaaaaggg gggactggaa gggctaattc 3720

actcccaacg aagacaaaat cgtcgagaga tgctgcatat aagcagctgc tttttgcttg 3780

tactgggtct ctctggttag accagatctg agcctgggag ctctctggct aactagggaa 3840

cccactgctt aagcctcaat aaagcttgcc ttgagtgctt caagtagtgt gtgcccgtct 3900

gttgtgtgac tctggtaact agagatccct cagacccttt tagtcagtgt ggaaaatctc 3960

tagcagtgta gtagtt 3976